# EUROPÄISCHE PATENTSCHRIFT

(11) **EP 0 804 146 B1**
(45) Veröffentlichungstag und Bekanntmachung des Hinweises auf die Patenterteilung: **29.09.1999**
(21) Anmeldenummer: 95913089.9
(22) Anmeldetag: 13.03.1995
(51) Int. Cl.: A61K 7/48, A61K 7/06, C11C 3/06, C08G 65/48

(54) **POLYGLYCERINPOLYRICINOLEATE**
POLYGLYCERINE POLYRICINOLEATES
POLYRICINOLEATES DE POLYGLYCERINE

(30) Priorität: 21.03.1994 DE 4409569
(43) Veröffentlichungstag der Anmeldung: 05.11.1997
(73) Patentinhaber: Henkel Kommanditgesellschaft auf Aktien, 40191 Düsseldorf (DE)
(72) Erfinder: ANSMANN, Achim, D-40699 Erkrath (DE); KAWA, Rolf, D-40789 Monheim (DE); GONDEK, Helga, D-40589 Düsseldorf (DE); STRAUSS, Gabriele, D-40589 Düsseldorf (DE); von Kries, Rainer, D - 89257 Illertissen (DE)
(86) Internationale Anmeldenummer: EP9500915
(87) Internationale Veröffentlichungsnummer: WO9525502

(56) Entgegenhaltungen:
- EP-A- 0 174 377
- EP-A- 0 579 159
- DE-A- 4 005 819
- DATABASE WPI Derwent Publications Ltd., London, GB; AN 89-259061(36) & JP,A,1 187 051 (DAIICHI KOGYO SEIYAKU) 26. Juli 1989
- DATABASE WPI Derwent Publications Ltd., London, GB; AN 84-002815(01) & JP,A,58 198 243 (ASAHI DENKA KOGYO) 18. November 1983

## Beschreibung

Die Erfindung betrifft neue Polyglycerinpolyricinoleate, erhältlich durch Veresterung von Polyricinolsäure mit technischem Polyglycerin einer definierten Zusamensetzung, ein Verfahren zu ihrer Herstellung, Mittel, die diese Stoffe enthalten sowie die Verwendung der neuen Polyglycerinpolyricinoleate als W/O-Emulgatoren.

### Stand der Technik

Polyglycerinpolyricinoleate sind seit langem als W/O-Emulgatoren bekannt und können zur Formulierung von niedrigviskosen W/O- Emulsionen eingesetzt werden [vgl. **EP-A1 0559013** (Th. Goldschmidt), **EP-A1 0440203** (Lotte Co.) und **WO 85/04346** (Meiji Milk Prods.)]. Es zeigt sich jedoch, daß Polyglycerinpolyrinoleate des Marktes nicht mit allen in der Kosmetik üblicherweise eingesetzten Öle Emulsionen bilden, sondern nur mit solchen eines bestimmten Polaritätsbereiches; zudem sind diese Emulsionen zudem nur eingeschränkt lagerstabil.

Die Aufgabe der Erfindung hat nun darin bestanden, neue W/O-Emulgatoren zur Verfügung zu stellen, die mit einem breiten Spektrum von Ölkörpern niedrigviskose und lagerstabile Emulsionen ergeben.

### Beschreibung der Erfindung

Gegenstand der Erfindung sind Polyglycerinpolyricinoleate, die man dadurch erhält, daß man Polyricinolsäure mit einem Eigenkondensationsgrad im Bereich von 2 bis 10 mit einem Polyglyceringemisch der Zusammensetzung

| | |
|---|---|
| Glycerin | 5 bis 35 (15 bis 25) Gew.-% |
| Diglycerine | 15 bis 40 (20 bis 32) Gew.-% |
| Triglycerine | 10 bis 35 (15 bis 25) Gew.-% |
| Tetraglycerine | 8 bis 20 (10 bis 18) Gew.-% |
| Pentaglycerine | 3 bis 10 ( 5 bis 9) Gew.-% |
| Oligoglycerine | ad 100 Gew.-% |

in an sich bekannter Weise verestert; in Klammern angegeben sind die bevorzugten Bereiche.

Überraschenderweise hat sich gezeigt, daß die Zusammensetzung der Polyglycerinkomponente entscheidenden Einfluß auf die anwendungstechnischen Eigenschaften der Emulgatoren hat. Die Erfindung schließt die Erkenntnis ein, daß Polyglycerinpolyricinoleate, die weniger als 5 oder mehr als 35 Gew.-% Glycerin in ihrer Polyglycerinkomponente enthalten, über ein signifikant schlechteres Emulgiervermögen verfügen.

Ein weiterer Gegenstand der Erfindung betrifft ein Verfahren zur Herstellung von Polyglycerinpolyricinoleaten, bei dem man Polyricinolsäure mit einem Eigenkondensationsgrad im Bereich von 2 bis 10 mit einem Polyglyceringemisch der Zusammensetzung

| | |
|---|---|
| Glycerin | 5 bis 35 (15 bis 25) Gew.-% |
| Diglycerine | 15 bis 40 (20 bis 32) Gew.-% |
| Triglycerine | 10 bis 30 (15 bis 25) Gew.-% |
| Tetraglycerine | 8 bis 20 (10 bis 18) Gew.-% |
| Pentaglycerine | 3 bis 10 ( 5 bis 9) Gew.-% |
| Oligoglycerine | ad 100 Gew.-% |

in an sich bekannter Weise verestert; in Klammern angegeben sind die bevorzugten Bereiche.

### Herstellung der Polyglycerinpolyricinoleate

Die Herstellung der Polyglycerinpolyricinoleate kann in an sich bekannter Weise erfolgen. Vorzugsweise wird dabei zunächst das Polyglycerin und dann die Polyricinolsäure hergestellt und schließlich beide kondensiert.

Die Herstellung eines Polyglycerins der oben genannten Zusammensetzung kann durch Eigenkondensation von Glycerin in Gegenwart von geeigneten Katalysatoren beispielsweise Silicaten gemäß **DE-A1 4029323** (Henkel) oder Boraten gemäß **DE-A1 4117033** (Henkel) bei Temperaturen im Bereich von 200 bis 260°C durchgeführt werden.

Die Herstellung der Polyricinolsäure erfolgt beispielsweise durch kontrollierte thermische Polykondensation von Ricinusölfettsäure, die zu etwa 90 Gew.-% aus Ricinolsäure besteht. Vorzugsweise werden dabei lineare Veresterungsprodukte mit 2 bis 10 und insbesondere 4 bis 6 Fettsäureeinheiten gebildet.

Bei der nachfolgenden Kondensation des technischen Polyglycerins mit der Polyricinolsäure wird eine komplexe Mischung homologer Polyester gebildet. Die Anteile an Mono-, Di-, Tri- und Oligoestern in den erfindungsgemäßen Polyglycerinpolyricinoleaten richtet sich nach den Einsatzverhältnissen der Ausgangsverbindungen. In einer bevorzugten Ausführungsform des erfindungsgemäßen Verfahrens wird ein Polyglycerinpolyricinoleat mit besonders vorteilhaften anwendungstechnischen Eigenschaften erhalten, indem man etwa 1000 kg Ricinusölfettsäure solange einer Eigenkondensation unterwirft, bis ein Produkt mit einer Säurezahl im Bereich von 50 bis 55 resultiert und dieses dann mit etwa 150 kg technischem Polyglycerin der oben angegebenen Zusammensetzung weiter kondensiert, bis die Säurezahl bis auf einen Wert kleiner 2 abgenommen hat.

### Kosmetische und pharmazeutische Zubereitungen

Ein weiterer Gegenstand der Erfindung betrifft kosmetische und/oder pharmazeutische Mittel mit einem Gehalt der neuen Polyglycerinpolyricinoleate.

Als Hilfs- und Zusatzstoffe kommen ferner Ölkörper, Co-Emulgatoren, Fette und Wachse, Stabilisatoren, Verdickungsmittel, biogene Wirkstoffe, Filmbildner, Duftstoffe, Farbstoffe, Perlglanzmittel, Konservierungsmittel, UV-Filter, Pigmente, Elektrolyte (z.B. Magnesiumsulfat) und pH-Regulatoren in Frage.en.

Als **Ölkörper** kommen beispielsweise aliphatische Kohlenwasserstoffe. Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₁₀-C₁₈-Fettalkoholen, Ester von linearen C₁₀-C₁₈-Fettsäuren mit verzweigten Alkoholen, insbesondere 2-Ethylhexanol, Ester von linearen und/oder verzweigten Fettsäuren mit zweiwertigen Alkoholen und/oder Guerbetalkoholen, Triglyceride auf Basis C₆-C₁₀-Fettsäuren, pflanzliche Öle, verzweigte primäre Alkohole, substituierte Cyclohexane, Guerbetcarbonate und/oder Dialkylether in Betracht. Von besonderer Bedeutung ist hierbei, daß sich die erfindungsgemäßen Polyglycerinpolyricinoleate zur Bildung von Emulsionen unter Verwendung sowohl von polaren, als auch mittel- oder unpolaren Ölkörpern mit Dipolmomenten in einem Bereich von kleiner 1 bis größer 2,5 Debye eignen.

Als **Co-Emulgatoren** kommen vorzugsweise bekannte W/O- und daneben auch O/W-Emulgatoren wie etwa Polyglycerinester, Sorbitanester oder teilveresterte Glyceride in Frage. Typische Beispiele für **Fette** sind Glyceride, als **Wachse** kommen u.a. Bienenwachs, Paraffinwachs oder Mikrowachse gegebenenfalls in Kombination mit hydrophilen Wachsen in Frage. Als **Stabilisatoren** können Metallsalze von Fettsäuren wie z.B. Magnesium-, aluminium und/oder Zinkstearat eingesetzt werden. Geeignete **Verdickungsmittel** sind beispielsweise vernetzte Polyacrylsäuren und deren Derivate, Polysaccharide, insbesondere Xanthan-Gum, Guar-Guar, Agar-Agar, Alginate und Tylosen, Carboxymethylcellulose und Hydroxyethylcellulose, ferner Fettalkohole, Monoglyceride und Fettsäuren, Polyacrylate, Polyvinylalkohol und Polyvinylpyrrolidon. Unter **biogenen Wirkstoffen** sind beispielsweise Pflanzenextrakte, Eiweißhydrolysate und Vitaminkomplexe zu verstehen. Gebräuchliche Filmbildner sind beispielsweise Hydrocolloide wie Chitosan, mikrokristallines Chitosan oder quaterniertes Chitosan, Polyvinylpyrrolidon, Vinylpyrrolidon-Vinylacetat-Copolymerisate, Polymere der Acrylsäurereihe, quaternäre Cellulose-Derivate und ähnliche Verbindungen. Als **Konservierungsmittel** eignen sich beispielsweise Formaldehydlösung, p-Hydroxybenzoat oder Sorbinsäure. Als **Perlglanzmittel** kommen beispielsweise Glycoldistearinsäureester wie Ethylenglycoldistearat, aber auch Fettsäuren und Fettsäuremonoglycolester in Betracht. Als **Farbstoffe** können die für kosmetische Zwecke geeigneten und zugelassenen Substanzen verwendet werden, wie sie beispielsweise in der Publikation "**Kosmetische Färbemittel**" **der Farbstoffkommission der Deutschen Forschungsgemeinschaft, veröffentlicht im Verlag Chemie**, **Weinheim**, **1984**, zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 80, vorzugsweise 5 bis 40 Gew.-% und der nicht wäßrige Anteil ("Aktivsubstanz") 20 bis 80, vorzugsweise 30 bis 70 Gew.-% - bezogen auf die Mittel - betragen. Die Herstellung der Mittel kann in an sich bekannter Weise, d.h. beispielsweise durch Heiß-, Kalt-, Heiß-Heiß/Kalt- bzw. PIT-Emulgierung erfolgen. Hierbei handelt es sich um ein rein mechanisches Verfahren, eine chemische Reaktion findet nicht statt.

### Gewerbliche Anwendbarkeit

Die erfindungsgemäßen Polyglycerinpolyricinoleate zeichnen sich durch ein verbessertes Emulgiervermögen aus. Die resultierenden Emulsionen sind vorzugsweise niedrigviskos und besitzen eine gegenüber den Produkten des Stands der Technik höhere Lager- und insbesondere Wärmestabilität.

Ein weiterer Gegenstand der Erfindung betrifft daher die Verwendung der erfindungsgemäßen Polyglycerinpolyricinoleate als W/O-Emulgatoren für die Herstellung kosmetischer und/oder pharmazeutischer Zubereitungen wie z.B. Hautcremes, Körperlotionen, Sonnenschutzmittel und dergleichen, in denen sie in Konzentrationen von 1 bis 20, vorzugsweise 2 bis 10 Gew.-% - bezogen auf die Mittel - enthalten sein können.

### Beispiele

### I. Eingesetzte Polyglycerinpolyricinoleate

Die Zusammensetzung der Polyglycerinkomponente ist in Tabelle 1 wiedergegeben. Die Emulgatoren wiesen bezüglich der Polyricinolsäurekomponente einen Kondensationsgrad von 5 auf.

**Tabelle 1**

| Zusammensetzung Polyglycerinpolyricinoleate | | |
|---|---|---|
| | **Emulgator A Gew.-%** | **Emulgator B Gew.-%** |
| Glycerin | 20 | 0 |
| Diglycerine | 30 | 21 |
| Triglycerine | 20 | 22 |
| Tetraglycerine | 15 | 20 |
| Pentaglycerine | 5 | 11 |
| Oligoglycerine | 10 | 26 |

Emulgator A ist erfindungsgemäß, Emulgator B stellt ein Vergleichsprodukt des Marktes dar.

### II. Anwendungstechnische Untersuchungen

Die Eigenschaften der Emulgatoren A und B wurden in verschiedenen W/O-Emulsionen gemäß Tabelle 2a und 2b getestet. Die Rezepturen R1 bis R4 sind erfindungsgemäß, die Rezepturen R5 bis R8 dienen dem vergleich. Myritol^{(R)} 318 stellt ein C₈₋₁₀-Triglycerid, Cetiol^{(R)} V Decyloleat und Cetiol^{(R)} SN Cetearylisononanoat dar. Die Viskosität der Produkte wurde nach Lagerung (1 Tag bzw. 1 Woche) nach Brookfield, RVF, 23°C, Spindel 5, 10 UpM bestimmt. Die Ergebnisse sind in Tabelle 3 zusammengestellt.

**Tabelle 2a**

| Eingesetzte erfindungsgemäße Rezepturen | | | | |
|---|---|---|---|---|
| | **R1 %** | **R2 %** | **R3 %** | **R4 %** |
| Emulgator A | 7,0 | 7,0 | 7,0 | 7,0 |
| Paraffinöl, dünnflüssig | 20,0 | - | - | - |
| Myritol 318 | - | 20,0 | - | - |
| Cetiol V | - | - | 20,0 | - |
| Cetiol SN | - | - | - | 20,0 |
| Glycerin, 86 Gew.-%ig | 5,0 | 5,0 | 5,0 | 5,0 |
| MgSO₄ * 7H₂O | 0,5 | 0,5 | 0,5 | 0,5 |
| Formaldehyd | 0,1 | 0,1 | 0,1 | 0,1 |
| Wasser | ad 100 % | | | |

**Tabelle 2b**

| Eingesetzte Vergleichsrezepturen | | | | |
|---|---|---|---|---|
| | **R5 %** | **R6 %** | **R7 %** | **R8 %** |
| Emulgator B | 7,0 | 7,0 | 7,0 | 7,0 |
| Paraffinöl, dünnflüssig | 20,0 | - | - | - |
| Myritol 318 | - | 20,0 | - | - |
| Cetiol V | - | - | 20,0 | - |
| Cetiol SN | - | - | - | 20,0 |
| Glycerin, 86 Gew.-%ig | 5,0 | 5,0 | 5,0 | 5,0 |
| MgSO₄ * 7H₂O | 0,5 | 0,5 | 0,5 | 0,5 |
| Formaldehyd | 0,1 | 0,1 | 0,1 | 0,1 |
| Wasser | ad 100 % | | | |

**Tabelle 3**

| Anwendungstechnische Ergebnisse | | | |
|---|---|---|---|
| **Rezeptur** | **Viskosität [mPa*s]** | | **Bemerkungen** |
| | **nach 1d** | **nach 1w** | |
| R1 | 24800 | 24000 | auch nach Lagerung über einen Zeitraum > 1 w stabile Emulsionen |
| R2 | 22800 | 22000 | |
| R3 | 16000 | 15200 | |
| R4 | 30000 | 29000 | |
| R5 | - | - | keine Emulsionsbildung |
| R6 | - | - | separiert nach 1 d |
| R7 | 16000 | 19000 | instabile Emulsion |
| R8 | - | - | separiert nach 1 d |

Die Beispiele zeigen, daß nur bei Verwendung der erfindungsgemäßen Polyglycerinpolyricinoleate zuverlässig, d.h. über ein breites Spektrum zu emulgierender Olkörper, stabile und niedrigviskose W/O-Emulsionen erhalten werden.

## Patentansprüche

1. Polyglycerinpolyricinoleate, dadurch erhältlich, daß man Polyricinolsäure mit einem Eigenkondensationsgrad im Bereich von 2 bis 10 mit einem Polyglyceringemisch der Zusammensetzung
| | |
|---|---|
| Glycerin | 5 bis 35 Gew.-% |
| Diglycerine | 15 bis 40 Gew.-% |
| Triglycerine | 10 bis 30 Gew.-% |
| Tetraglycerine | 8 bis 20 Gew.-% |
| Pentaglycerine | 3 bis 10 Gew.-% |
| Oligoglycerine | ad 100 Gew.-% |
in an sich bekannter Weise verestert.

2. Verfahren zur Herstellung von Polyglycerinpolyricinoleaten, bei dem man Polyricinolsäure mit einem Eigenkondensationsgrad im Bereich von 2 bis 10 mit einem Polyglyceringemisch der Zusammensetzung
| | |
|---|---|
| Glycerin | 5 bis 35 Gew.-% |
| Diglycerine | 15 bis 40 Gew.-% |
| Triglycerine | 10 bis 30 Gew.-% |
| Tetraglycerine | 8 bis 20 Gew.-% |
| Pentaglycerine | 3 bis 10 Gew.-% |
| Oligoglycerine | ad 100 Gew.-% |
in an sich bekannter Weise verestert.

3. Kosmetische und/oder pharmazeutische Zubereitungen, enthaltend Polyglycerinpolyricinoleate nach Anspruch 1.

4. Verwendung von Polyglycerinpolyricinoleaten nach Anspruch 1 als W/O-Emulgatoren für kosmetische und/oder pharmazeutische Zubereitungen.

## Claims

1. Polyglycerol polyricinoleates obtainable by esterifying polyricinoleic acid having a degree of autocondensation of 2 to 10 with a polyglycerol mixture of the following composition:
| | |
|---|---|
| glycerol | 5 to 35% by weight |
| diglycerols | 15 to 40% by weight |
| triglycerols | 10 to 30% by weight |
| tetraglycerols | 8 to 20% by weight |
| pentaglycerols | 3 to 10% by weight |
| oligoglycerols | to 100 % by weight |
by methods known per se.

2. A process for the production of polyglycerol polyricinoleates, in which polyricinoleic acid having a degree of autocondensation of 2 to 10 is esterified with a polyglycerol mixture of the following composition:
| | |
|---|---|
| glycerol | 5 to 35% by weight |
| diglycerols | 15 to 40% by weight |
| triglycerols | 10 to 30% by weight |
| tetraglycerols | 8 to 20% by weight |
| pentaglycerols | 3 to 10% by weight |
| oligoglycerols | to 100 % by weight |
by methods known per se.

3. Cosmetic and/or pharmaceutical formulations containing the polyglycerol polyricinoleates claimed in claim 1,

4. The use of the polyglycerol polyricinoleates claimed in claim 1 as w/o emulsifiers for cosmetic and/or pharmaceutical formulations.

## Revendications

1. Polyricinoléates de polyglycérine accessibles en ce qu'
on estérifie d'une manière connue en soi de l'acide polyricinoléique ayant un degré d'autocondensation dans la zone de 2 à 10 avec un mélange de polyglycérols de composition :
| | |
|---|---|
| glycérol | 5 à 35 % en poids |
| diglycérol | 15 à 40 % en poids |
| triglycérol | 10 à 30 % en poids |
| tétraglycérol | 8 à 20 % en poids |
| pentaglycérol | 3 à 10 % en poids |
| oligoglycérol | qsp 100 % en poids |

2. Procédé de préparation de polyricinoléates de polyglycérine
dans lequel
on estérifie d'une manière connue en soi de l'acide polyricinoléique ayant un degré d'autocondensation dans la zone de 2 à 10 avec un mélange de polyglycérols de composition :
| | |
|---|---|
| glycérol | 5 à 35 % en poids |
| diglycérol | 15 à 40 % en poids |
| triglycérol | 10 à 30 % en poids |
| tétraglycérol | 8 à 20 % en poids |
| pentaglycérol | 3 à 10 % en poids |
| oligoglycérol | qsp 100 % en poids |

3. Préparations cosmétiques et/ou pharmaceutiques renfermant des polyricinoléates de polyglycérine selon la revendication 1.

4. Utilisation des polyricinoléates de polyglycérine selon la revendication 1 en tant qu'agents émulsionnants W/O pour des préparations cosmétiques et/ou pharmaceutiques.
